# EUROPEAN PATENT SPECIFICATION

(11) **EP 4 093 291 B1**
(45) Date of publication and mention of the grant of the patent: **17.09.2025**
(21) Application number: 21703151.7
(22) Date of filing: 21.01.2021
(51) Int. Cl.: A61B 8/00, A61B 5/24, A61B 8/08

(54) **WEARABLE ULTRASOUND APPARATUS**
TRAGBARE ULTRASCHALLVORRICHTUNG
DISPOSITIF À ULTRASONS PORTABLE

(30) Priority: 24.01.2020 US 202062965276 P
(43) Date of publication of application: 30.11.2022
(73) Proprietor: Dawako Medtech, S.L., 46980 Paterna (Valencia) (ES)
(72) Inventor: MACIA BARBER, Agustin, 46980 Paterna (Valencia) (ES); GARCIA CHOCANO, Victor Manuel, 46980 Paterna (Valencia) (ES); GODOY, Eduardo Jorge, 46980 Paterna (Valencia) (ES)
(74) Representative: Grund, Martin
(86) International application number: PCT/EP2021/051377
(87) International publication number: WO 2021/148557

(56) References cited:
- WO-A1-2013/130979
- US-A1- 2019 269 942

## Description

### RELATED APPLICATIONS

This application claims priority and is entitled to the filing date of U.S. provisional application serial number 62/965,276, filed on January 24, 2020.

### BACKGROUND

The subject of this patent application relates generally to ultrasound devices, and more particularly to a wearable ultrasound apparatus configured for use in connection with various biomedical applications.

By way of background, ultrasound waves are utilized in many different fields, typically as a tool to penetrate a medium to measure its reflection signature. In medicine, ultrasound imaging devices are usually used for diagnostic medical imaging of internal organs, muscles, tendons and other objects positioned within a patient, among other applications. Traditional ultrasound imaging devices are capable of providing sophisticated live images and enable extraction of characteristic features using advanced signal processing techniques. However, they are generally large, stationary and expensive. Moderate size imaging devices with limited mobility, such as computer-on-wheels systems, are also available with performance generally similar to the larger systems. Handheld versions, along with wearable versions, of such devices have also been developed in recent years, which provide relatively more mobility. However, to Applicant's knowledge, none of these known devices are capable of simultaneously acquiring ultrasonic imaging, electrophysiological, hemodynamic, and metabolic information of internal organs, muscles, tendons and other soft tissues of the patient in biomedical and clinical applications.

US 2019/269942 A1 and WO 2013/130979 A1 disclose wearable ultrasound apparatuses comprising an ultrasound module and an electrophysiological module.

Aspects of the present invention fulfill these needs and provide further related advantages as described in the following summary.

It should be noted that the above background description includes information that may be useful in understanding aspects of the present invention. It is not an admission that any of the information provided herein is prior art or relevant to the presently claimed invention, or that any publication specifically or implicitly referenced is prior art.

### SUMMARY

Aspects of the present invention teach certain benefits in construction and use which give rise to the exemplary advantages described below.

The invention is defined by the appended claims.

The present invention solves the problems described above by providing a wearable ultrasound apparatus configured for use in connection with various biomedical applications, including musculoskeletal ("MSK") imaging and analysis. The apparatus provides an ultrasound module configured for obtaining at least one ultrasound image of a portion of a user's body on which the at least one ultrasound module is positioned (hereinafter referred to as the "target site" for simplicity purposes), an electrophysiological ("EP") module configured for detecting bioelectric signals of the target site, and a near-infrared spectroscopy ("NIRS") module configured for monitoring oxygenation status and/or biochemical measurements of the target site. The ultrasound module comprises at least one ultrasound transducer positioned on at least one resilient substrate, the at least one ultrasound transducer comprising at least one sensor; at least one conductive layer positioned in electrical communication with the at least one sensor; and at least one ultrasound transceiver in electrical communication with the at least one sensor. The EP module is positioned on the at least one resilient substrate. The apparatus further comprises at least one controller in electrical communication with each of the ultrasound module and EP module via the conductive layers, the at least one controller configured for selectively causing the ultrasound module to obtain at least one ultrasound image of the target site upon detection of bioelectric signal in the target site via the EP module. The NIRS module is positioned on the at least one resilient substrate, in electrical communication with the at least one controller.

Other features and advantages of aspects of the present invention will become apparent from the following more detailed description, taken in conjunction with the accompanying drawings, which illustrate, by way of example, the principles of aspects of the invention.

### BRIEF DESCRIPTION OF THE DRAWINGS

The accompanying drawings illustrate aspects of the present invention. In such drawings:
Figure 1 is a diagrammatic cross-sectional view of an exemplary wearable ultrasound apparatus, in accordance with at least one embodiment;
Figures 2A, 2B and 2C are diagrammatic views of exemplary piezoelectric sensors and electrodes of an exemplary ultrasound transducer, in accordance with at least one embodiment;
Figure 3 is a further diagrammatic view of exemplary piezoelectric sensors, in accordance with at least one embodiment;
Figure 4 is a further diagrammatic view of exemplary array of piezoelectric sensors, in accordance with at least one embodiment;
Figure 4A is a detailed view of the section defined by line 4A of Figure 4;
Figures 5A and 5B are diagrammatic views of exemplary arrays of piezoelectric sensors, in accordance with at least one embodiment;
Figure 6 is a schematic view of an exemplary wearable ultrasound apparatus, in accordance with at least one embodiment;
Figure 7 is a schematic view of an exemplary ultrasound transceiver, in accordance with at least one embodiment;
Figure 8 is a schematic view of an exemplary electrophysiological ("EP") module, in accordance with at least one embodiment;
Figure 9 is a schematic view of an exemplary near-infrared spectroscopy ("NIRS") module, in accordance with at least one embodiment;
Figure 10 is a diagrammatic cross-sectional view of a further exemplary wearable ultrasound apparatus, in accordance with at least one embodiment; and
Figure 11 is a diagrammatic cross-sectional view of a still further exemplary wearable ultrasound apparatus, in accordance with at least one embodiment.

The above described drawing figures illustrate aspects of the invention in at least one of its exemplary embodiments, which are further defined in detail in the following description. Features, elements, and aspects of the invention that are referenced by the same numerals in different figures represent the same, equivalent, or similar features, elements, or aspects, in accordance with one or more embodiments.

### DETAILED DESCRIPTION

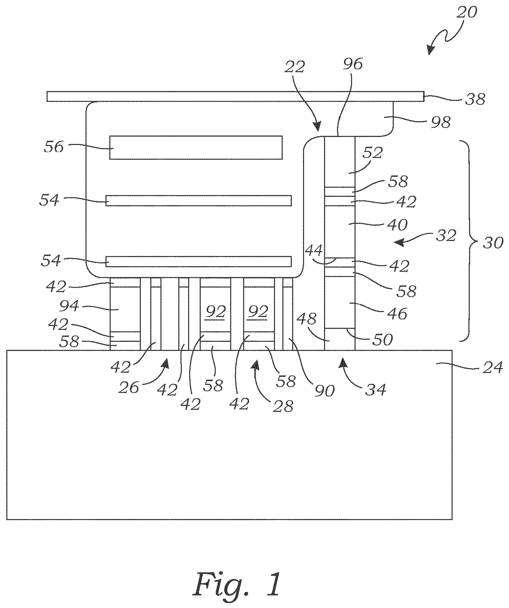

Turning now to Fig. 1, there is shown a diagrammatic cross-sectional view of an exemplary embodiment of a wearable ultrasound apparatus **20** configured for use in connection with various biomedical applications, including musculoskeletal ("MSK") imaging and analysis. The apparatus 20 provides an ultrasound module **22** configured for obtaining at least one ultrasound image of a portion of a user's body on which the at least one ultrasound module **22** is positioned (hereinafter referred to as the "target site" **24** for simplicity purposes), a electrophysiological ("EP") module **26** configured for detecting bioelectric signals of the target site **24,** and a near-infrared spectroscopy ("NIRS") module **28** configured for monitoring oxygenation status and/or biochemical measurements of the target site **24,** each of which are discussed further below. At the outset, it should be noted that the particular arrangement of components illustrated in Fig. 1 is merely exemplary. Thus, in further embodiments, as discussed further below, the various components may take on a number of other arrangements.

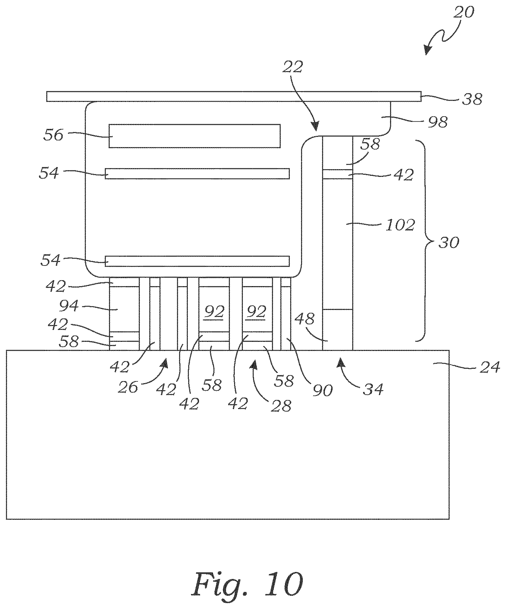

In at least one embodiment, the ultrasound module **22** provides at least one ultrasound transducer 30 intended to operate in the range of 7-14 MHz for superficial scans and 2-6 MHz for deeper targets. However, in further embodiments, the at least one ultrasound transducer **30** may operate in any other range, now known or later developed, capable of allowing the apparatus **20** to substantially carry out the functionality described herein. Additionally, in at least one embodiment, the at least one ultrasound transducer **30** is configured for operating in a pulse-echo configuration - i.e., it is configured to emit and subsequently receive ultrasonic pulses in order to obtain the at least one ultrasound image. In at least one embodiment, the at least one ultrasound transducer **30** comprises at least one piezoelectric sensor **32.** In at least one such embodiment, the ultrasound transducer **30** comprises a plurality of piezoelectric sensors **32** configured as at least one array **34,** with the quantity of piezoelectric sensors **32** in a given array **34** ranging between 2 and 256. However, in further embodiments, any other quantities of piezoelectric sensors **32** may be utilized. In at least one alternate embodiment, as illustrated in Fig. 10, the at least one ultrasound transducer **30** comprises at least one microelectromechanical ("MEM") sensor **102** - such as at least one capacitive micromachined ultrasonic transducer ("CMUT") or piezoelectric micromachined ultrasonic transducer ("PMUT"), for example - either in addition to or in lieu of the at least one piezoelectric sensor **32.** Both CMUT and PMUT are based on the oscillation of a membrane suspended on a cavity developed in a silicon substrate.

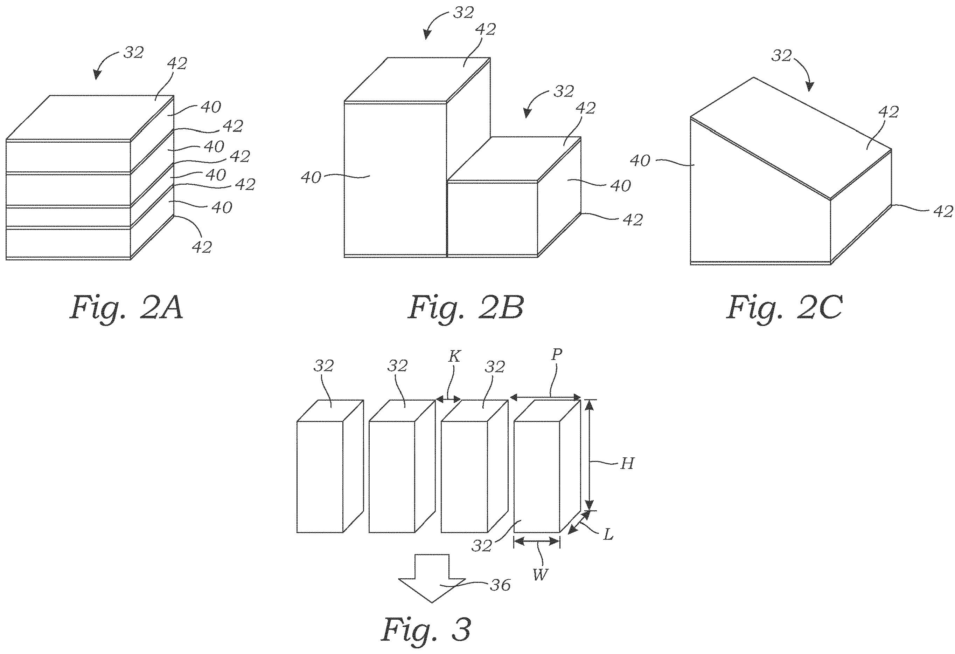

The at least one ultrasound transducer **30** is positioned on at least one resilient substrate **38.** In at least one such embodiment, the resilient substrate **38** is comprised of a material that is flexible and/or stretchable. For example, in at least one such embodiment, the material is comprised of at least one of a silicon-based material, rubber, thermoplastic elastomers, polymeric materials, foils (such as those mixed with epoxy), and various fabrics. In at least one further embodiment, the resilient substrate **38** is comprised of a material that is transparent, flexible, and conformable in nature. Additionally, in at least one embodiment, the material is biocompatible, latex-free, non-toxic, and non-allergenic. In still further embodiments, the resilient substrate **38** may comprise any other materials (or combinations of materials) having flexible and/or rigid-flexible characteristics, now known or later developed, so long as the apparatus **20** is capable of substantially carrying out the functionality described herein. In at least one embodiment, the resilient substrate **38** is made of composite epoxy material ("CEM"), fiberglass, or paper base class materials providing a solid foundation for printed circuit boards ("PCBs"). For example, in at least one such embodiment, the material is comprised of at least one of an epoxy resin (FR4, FR5, FE-3), PF resin (XPC, FR1, FR2), and polyester resin. In at least one alternate embodiment, the resilient substrate **38** is positioned and employed to perform the function of any of the layers of the ultrasound module **22,** EP module **26** or NIRS module **28,** provided that it is placed at the position of the layer whose function performs and its material properties are suitable. In at least one embodiment, the resilient substrate **38** has a thickness of approximately 180 micrometers or less, such that the apparatus **20** has an overall thickness of approximately 25 millimeters or less. However, in further embodiments, the resilient substrate **38** may have any other thickness, so long as the apparatus **20** is capable of substantially carrying out the functionality described herein. In at least one embodiment, the resilient substrate **38** is configured as a flexible film with signal traces embedded within or over the resilient substrate **38,** and the at least one piezoelectric sensor **32** is attached to the resilient substrate **38.** In at least one alternate embodiment, the at least one piezoelectric sensor **32** and the signal traces are simultaneously screen-printed onto the resilient substrate **38,** which can provide a number of benefits. For example, in at least one such embodiment, simultaneously screen-printing the at least one piezoelectric sensor **32** and the signal traces onto the resilient substrate **38** can reduce the number of steps involved in the manufacturing process. Additionally, the size, shape and arrangement of the at least one piezoelectric sensor **32** (relative to the resilient substrate **38)** can be freely customized, depending on the intended use of the apparatus **20** in a given embodiment. Specifically, the shape of the at least one piezoelectric sensor **32** can be modified to have rounded corners, so as to introduce aperture apodization, thereby improving sidelobe suppression. Further exemplary shapes could include (but are in no way limited to) rings, hexagons, circles, rectangles, etc. Additionally, in at least one such embodiment, where the at least one piezoelectric sensor **32** comprises a piezoelectric material **40** sandwiched between two or more electrodes **42** (as illustrated in Figs. 2A, 2B and 2C, and discussed further below), given that a distance between electrodes **42** (based at least partially on the thickness of the piezoelectric material **40)** defines an excitation frequency therebetween, a plurality of electrodes **42** can be implemented with varying distances therebetween so as to enable the at least one ultrasound transducer **30** to operate at multiple frequencies and with improved bandwidth.

In at least one embodiment, the distance between centers of two contiguous piezoelectric sensors **32** of an array **34** is less than approximately 0.5λ for phased array operation and approximately 0.75λ-3λ for linear array operation, where *λ =* c / *f,* with *λ* being the wavelength of the ultrasound signal with frequency *f* and longitudinal sound speed c≈1500 m/s. Some numeric examples of the aforementioned limits are given in Table 1 below.

**TABLE 1**

| | **7 MHz** | **10 MHz** | **14 MHz** |
|---|---|---|---|
| **0.5 λ** | 107.1 µm | 75 µm | 53.6 µm |
| **0.75 λ** | 160.7 µm | 112.5 µm | 80.4 µm |
| **3 λ** | 642.9 µm | 450 µm | 321.4 µm |

In at least one embodiment, as illustrated in Fig. 3, each of the piezoelectric sensors **32** has a width W that is relatively less than a pitch **P,** given that a small kerf **K** (i.e., a separation) is required between the piezoelectric sensors **32** so as to provide acoustic inter-element isolation to each of the piezoelectric sensors **32.** Additionally, in at least one embodiment, each of the piezoelectric sensors **32** has a thickness or height **H** that is at least partially dependent on the resonant frequency at which the ultrasound transducer **30** operates. Several examples of thickness are shown in Table 2 below for different materials. Additionally, in at least one embodiment, each of the piezoelectric sensors **32** has a length **L** which is less restricted by design constraints in comparison with the other two dimensions.

**TABLE 2**

| | **7 MHz** | **10 MHz** | **14 MHz** |
|---|---|---|---|
| PZT-5A | 230.5 µm | 161.3 µm | 115.3 µm |
| PZT-5H | 271.4 µm | 190 µm | 135.7 µm |
| PVDF | 157.1 µm | 110 µm | 78.6 µm |

In at least one embodiment, the thickness/height **H** of a given piezoelectric sensor **32** is a function of frequency of the sound waves. In at least one such embodiment, each of the piezoelectric sensors **32** has a height **H** of approximately 300 micrometers or less. However, in further embodiments, each of the piezoelectric sensors **32** may have any other height **H,** so long as the apparatus **20** is capable of substantially carrying out the functionality described herein.

In at least one embodiment, the at least one piezoelectric sensor **32** may be made of any suitable material, including but not limited to a flexible piezoelectric coating (film, paste or paint), a ceramic transducer, or a polymer block transducer. Additionally, in at least one embodiment, the at least one piezoelectric sensor **32** may be comprised of quartz, polyvinylidene fluoride, ceramic including PZT and screen-printed ceramic, magneto strictive, or composite material including molded ceramic and benders. For example, the piezoelectric material may be selected from the group consisting of Polyvinylidene fluoride (PVDF) and its co-polymers, lead zirconate titanate Pb(Zr,Ti)O3, lead metaniobate Pb(Nb2O6), modified lead titanate PbTi3, (Pb, Ca)Ti03, (Pb, Sm)Ti03, barium titanate BaTi03, PMN-PT(I-x)Pb(Mg1/2, Nb2/3)O 3-xPb-TiO3, PZN-PT/BT Pb(ZN1/2, Nb2/3)O3-x PbTiO3-BaTiO3, (I-x)Pb(ZN1/2, Nb2/3)O3-x(yPbTiO3-(l-y)PbZrO3). In at least one embodiment, the at least one piezoelectric sensor **32** is comprised of a flexible piezoelectric coating (film, paste or paint), such as PVDF or a co-polymer thereof. It will be appreciated to those skilled in the art that recent developments in flexible piezoelectric coatings, (such as U.S. Patent No. 10,079,336) provide a piezoelectric material **40** that can be applied on a variety of substrates. Of course, other flexible piezoelectric coatings may be utilized in at least one embodiment of the present invention.

As also illustrated in Fig. 2A, in at least one embodiment, multiple layers of piezoelectric material **40** may be stacked on one another, with electrodes **42** positioned therebetween. In at least one further embodiment, as illustrated in Fig. 2B, multiple piezoelectric sensors **32** may be positioned in a side-by-side arrangement. Another benefit with simultaneously screen-printing the at least one piezoelectric sensor **32** and the signal traces onto the resilient substrate **38** is the ability to integrate additional resources such as the EP module **26** and/or the NIRS module **28,** as discussed further below. Thus, the sizes, shapes, dimensions, configurations and quantities of each of the at least one piezoelectric sensor **32** and corresponding resilient substrate **38** as depicted in the drawings (and as described herein) are merely exemplary. In further embodiments, each of the at least one piezoelectric sensor **32** and corresponding resilient substrate **38** may take on any other size, shape, dimensions, configurations and/or quantities, now known or later developed, so long as the apparatus **20** is capable of substantially carrying out the functionality described herein. In still further embodiments, any other technique (or combinations of techniques) for positioning the at least one piezoelectric sensor **32** onto resilient substrate **38,** now known or later developed, may be substituted.

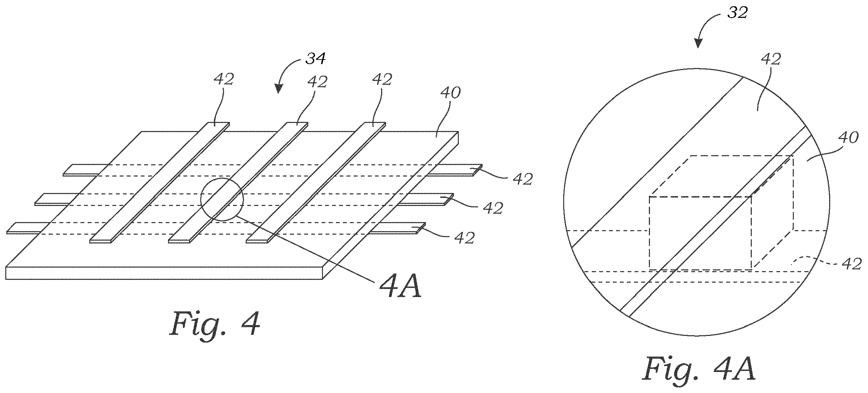

In at least one further embodiment, the piezoelectric material **40** may be sandwiched between a plurality of electrodes **42** which are arranged in a row-column configuration so as to form quasi-two-dimensional arrays. An example of such an embodiment is illustrated in Figs. 4 and 4A, in which the electrodes **42** are arranged in a 3x3 matrix, and the piezoelectric material **40** is positioned substantially between electrodes **42** in the areas where said electrodes **42** overlap (as illustrated in Fig. 4A).

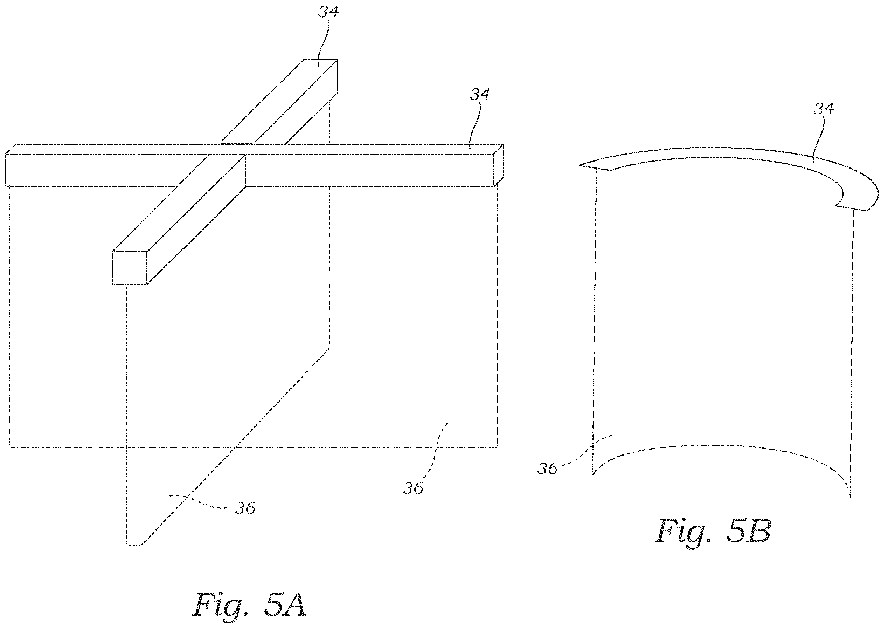

In at least one embodiment, the at least one ultrasound transducer **30** is configured to perform B-mode scans (or "B-scans") of the target site **24.** However, in further embodiments, the at least one ultrasound transducer **30** may be configured to perform other types of scans, now known or later developed, including but not limited to A-mode (or "amplitude mode"), C-mode, M-mode (or "motion mode"), Doppler mode, Pulse inversion mode, Harmonic mode, etc. The positioning of the at least one ultrasound transducer **30** is dependent upon the portion of the user's body that requires at least one ultrasound image. Additionally, as mentioned above, in at least one embodiment, the at least one ultrasound transducer **30** comprises a plurality of piezoelectric sensors **32** configured as at least one array **34.** In at least one such embodiment, the at least one array **34** may be arranged in a variety of configurations. For example, as illustrated in the diagram of Fig. 5A, two or more linear arrays **34** may be arranged so as to acquire orthogonal cross-sections of the target site **24.** As another example, as illustrated in the diagram of Fig. 5B, the at least one array **34** may be configured as a curve (rather than linear). As yet another example, the at least one array **34** may be configured as a convex curve, thereby providing a relatively wider field of view. Additionally, the size of the at least one array **34** is dependent, at least in part, on the specific context in which the apparatus **20** is to be used. As mentioned above, the quantity of piezoelectric sensors **32** in a given array **34** ranges between 2 and 256 in at least one embodiment. However, in further embodiments, any other quantities of piezoelectric sensors **32** may be utilized. Thus, the sizes, shapes, dimensions, configurations and quantities of the at least one array **34** as depicted in the drawings (and as described herein) are merely exemplary. In further embodiments, the at least one array **34** may take on any other size, shape, dimensions, configurations and/or quantities, now known or later developed, so long as the apparatus **20** is capable of substantially carrying out the functionality described herein.

In at least one embodiment, where the at least one ultrasound transducer **30** is configured to perform B-mode scans of the target site and comprises a plurality of piezoelectric sensors **32** configured as at least one linear array **34,** a subset of adjacent or contiguous piezoelectric sensors **32** are configured for being simultaneously excited/activated at any given time. The signals of each piezoelectric sensor **32** of the subset can be identical or present specific time delays to provide focusing or beam steering, while different signal amplitudes on each piezoelectric sensor **32** of the subset may be applied to achieve apodization. Multi-line acquisition techniques may be used to improve frame rate of the resulting at least one ultrasound image. In still further embodiments, multi-line transmission may be utilized in order to further improve frame rate, including the simultaneous excitation of multiple ultrasound beams of either the same or different frequencies. Additionally, in at least one embodiment harmonic imaging is implemented to improve image resolution.

In at least one embodiment, as illustrated in Fig. 1, a bottom surface **44** of the at least one piezoelectric sensor **32** provides at least one matching layer **46** configured for providing acoustic impedance adaption. When an acoustic wave **36** encounters a boundary between two layers having a relatively large variance in their respective acoustic impedances, the acoustic wave **36** is reflected at the boundary. Thus, in at least one embodiment, using a plurality of matching layers **46** enables the acoustic impedance of each matching layer **46** to be varied gradually to minimize reflections. In at least one such embodiment, the at least one matching layer **46** (or at least a bottom most one of the at least one matching layer **46)** is configured for selectively adhering the at least one ultrasound transducer **30** to the target site **24** - either directly (i.e., adhering to the user's skin) or indirectly (i.e., adhering to a garment or other material that, in turn, is in contact with the user's skin). In at least one embodiment, the quantity of matching layers **46** is dependent (at least in part) on the characteristics of the at least one piezoelectric sensor **32.** In general, it has been found that a higher quantity of matching layers **46** results in relatively better adaption (i.e., less energy is reflected to the at least one ultrasound transducer **30)** and broadband operation, which improves the axial resolution of the at least one ultrasound image). In at least one embodiment, where the at least one piezoelectric sensor **32** is screen-printed onto the corresponding at least one resilient substrate **38,** the at least one resilient substrate **38** itself may be configured to function as a matching layer **46.** In at least one embodiment, similar to the at least one resilient substrate **38,** the at least one matching layer **46** is comprised of a material that is flexible and/or stretchable. For example, in at least one such embodiment, the material is a silicone adhesive gel. In further embodiments, the material is at least one of rubber, silicone, thermoplastic elastomers or other polymeric material such as polyester, polyethylene terephthalate, polyethylene naphthalate, polycarbonate, polystyrene, polyacryl, polyether sulfone, etc. Additionally, in at least one embodiment, where the at least one matching layer **46** is configured for being in direct contact with the user's skin, the at least one matching layer **46** is biocompatible, latex-free, non-toxic, and non-allergenic. In still further embodiments, the at least one matching layer **46** may comprise any other appropriate materials (or combinations of materials) having flexible and/or stretchable characteristics, now known or later developed, capable of allowing the at least one matching layer **46** to substantially carry out the functionality described herein.

In at least one embodiment, where the at least one matching layer **46** is comprised of polymeric materials, the acoustic impedance of the polymeric materials may be increased by the incorporation of one or more fillers. Suitable fillers include, but are not limited to, PZT, tungsten, alumina, silica glass, tungsten carbide, titanium, glass powder and the like, with glass powder being preferred. In at least one such embodiment, the size of the filler particles are in the range of approximately 0.1-50 microns and preferably from approximately 0.5-5 microns. The amount of filler employed will be that amount necessary to impart the desired acoustic impedance. Normally, from about 2 to about 50 percent filler by volume and preferably from about 5 to about 30 percent filler by volume is employed. A preferred polymeric material is silicone rubber.

In at least one embodiment, as illustrated in Fig. 1, the ultrasound module **22** further provides a coupling layer **48** positioned in contact with a bottom surface **50** of the at least one matching layer **46** (or the bottom most one of the at least one matching layer **46)** and configured for selectively adhering the ultrasound module 22 (and, in turn, the apparatus 20) to the target site **24** - either directly (i.e., adhering to the user's skin) or indirectly (i.e., adhering to a garment or other material that, in turn, is in contact with the user's skin). In at least one embodiment, the coupling layer **48** comprises a sonolucent silicone gel or other adhesive material capable of transmitting ultrasound signals between the ultrasound module **22** and the target site **24.** By "sonolucent," it is meant that the gel is capable of transmitting ultrasound pulses without introducing significant interference or attenuation, such that an acceptable acoustic response can be obtained from the target site **24.** Thus, the coupling layer **48** materials may be selected for their ability to provide a robust and void-free contact between the ultrasound module **22** and the adjacent target site **24.** The acoustic impedance of the coupling layer **48** should be close to that of the adjacent target site **24** to provide impedance matching. In at least one embodiment, the coupling layer **48** is part of the matching layer **46** and its impedance is selected according to the design guidelines of such matching layer **46.** In at least one embodiment, where the at least one piezoelectric sensor **32** is screen-printed onto the corresponding at least one resilient substrate **38,** the at least one resilient substrate **38** itself may be configured to function as the coupling layer **48.** Additionally, in at least one embodiment, the coupling layer **48** provides a temporary backing configured for being peeled off prior to the coupling layer **48** being adhered to the target site **24.** In at least one embodiment, the coupling layer **48** has a thickness of approximately 100 to 500 micrometers (e.g., 100, 200, 300, 400, 500 micrometers or some range therebetween). However, in further embodiments, the coupling layer **48** may have any other thickness, so long as the apparatus 20 is capable of substantially carrying out the functionality described herein. Additionally, in at least one embodiment, where the at least one coupling layer **48** is configured for being in direct contact with the user's skin, the at least one coupling layer **48** is biocompatible, latex-free, non-toxic, and non-allergenic. In still further embodiments, the at least one coupling layer **48** may comprise any other appropriate materials (or combinations of materials) having the above described characteristics, now known or later developed, capable of allowing the at least one coupling layer **48** to substantially carry out the functionality described herein.

In at least one embodiment, as illustrated in Fig. 1, the ultrasound transducer 30 further provides at least one backing layer **52** positioned in contact with the at least one piezoelectric sensor **32** opposite the corresponding side at which energy is intended to be radiated (i.e., where the target site **24** is located) , such that the at least one piezoelectric sensor **32** is substantially sandwiched between the at least one backing layer **52** and the target site **24.** The at least one backing layer **52** is configured for absorbing any ultrasound waves radiated by the at least one piezoelectric sensor **32** that is not directed toward the target site **24,** thus preventing any reverberations and/or resonances that would otherwise decrease the bandwidth of the emitted pulses from the at least one piezoelectric sensor **32.** In at least one embodiment, where the at least one piezoelectric sensor **32** is screen-printed onto the corresponding at least one resilient substrate **38,** the at least one resilient substrate **38** itself may be configured to function as the backing layer **52.** Accordingly, in at least one embodiment, the at least one backing layer **52** is made of a material having an acoustic impedance close to the at least one piezoelectric sensor **32** and having a relatively high damping coefficient. In such embodiments, because the acoustic impedance of the at least one backing layer **52** is similar to that of the at least one piezoelectric sensor **32** and because of the absorption of the material of the at least one backing layer **52,** most of the backward transmitted wave quickly attenuates and become heat, and only a very small portion may bounce back. In at least one embodiment, the at least one backing layer **52** is constructed out of at least one of tungsten-loaded epoxy, pyrolytic, brass, carbon, etc. In still further embodiments, the at least one backing layer **52** may comprise any other appropriate materials (or combinations of materials), now known or later developed, capable of allowing the at least one backing layer **52** to substantially carry out the functionality described herein.

With continued reference to Fig. 1, in at least one embodiment, the ultrasound module **22** further provides a pair of conductive layers **54** positioned for substantially sandwiching the at least one piezoelectric sensor **32** therebetween, such that the conductive layers **54** integrate the electrodes **42** of each piezoelectric sensor **32** as well as the traces that interconnect them to the electronics system (hereinafter referred to as the "microelectronics module" **56,** as discussed further below) associated with the ultrasound module **22.** In at least one embodiment, the conductive layers **54** each comprises a thin metal film such as aluminum, copper, gold, molybdenum, iridium, magnesium, silver, lithium fluoride and alloys thereof, or a non-metal material. Additionally, in at least one embodiment, the thickness of each conductive layer **54** is typically about 200 µm or less (e.g., about 200, 180, 160, 140, 120, 100, 90, 80, 70, 60, 50, 40, 30 µm or less). Preferably, the thickness of each conductive layer **54** is less than 10 µm (e.g., about 10, 9, 8, 7, 6, 5, 4, 3, 2, 1, 0.8, 0.6, 0.4, 0.2 µm or less or some range therebetween). Additionally, in at least one embodiment, the conductive layers **54** are flexible. In at least one such embodiment, the conductive layers **54** are constructed out of transparent conductive polymer materials, such as indium tin oxide (ITO), fluorine-doped tin oxide (FTO), ZnO-Ga2O3, ZnO-Al2O3, SnO2-Sb2O3, and polythiophene. In addition, the conductive layers **54** may be comprised of silver or copper grids or bushbars plated on a transparent substrate or silver nanowires or nanoparticles deposited on a substrate with a poly(3,4-ethylenedioxythiophene) poly(styrenesulfonate) (PEDOT:PSS) coating. Additional conductive polymer layers may be added to improve conductivity. In at least one embodiment, the conductive layers **54** may be carbon-based, for example, carbon nanotubes ("CNT"), carbon nanowires, or graphene, and the like. One preferred conductive layer **54** (electrically conductive and transparent for infrared radiation) comprises graphene. While one or two layers of graphene is preferred, the conductive layers **54** may each comprise about 1 to 20 layers of graphene (e.g., 1, 2, 3, 4, 5, 6, 7, 8, 9, 10, 11, 12, 13, 14, 15, 16, 17, 18, 19, 20 layers or some range therebetween). In at least one embodiment, the conductive layers **54** may comprise several internal conductive layers **54** separated by insulating materials in order to manage a high number of tracks.

With continued reference to Fig. 1, in at least one embodiment, the ultrasound transducer **30** further provides a pair of encapsulation layers **58** positioned for substantially sandwiching the pair of conductive layers **54** (and, in turn, the at least one piezoelectric sensor **32)** therebetween, such that the encapsulation layers **58** are configured for isolating the at least one piezoelectric sensor **32** from the surrounding environment. In at least one embodiment, the encapsulation layers **58** are substantially impermeable to moisture and oxygen. In general, the moisture and oxygen sensitive components of the apparatus **20** should be enclosed by materials having gas permeation properties. The encapsulation layers **58** preferably achieve low water vapor permeation rates of 10⁻⁴ g/ni²/day or less, 10⁻⁵ g/m²/day or less, and even more preferably about 10⁻⁶ g/ni²/day or less. In at least one embodiment, the encapsulation layers **58** are constructed out of glass or a plastic, for example. In at least one embodiment, the encapsulation layers **58** are comprised of a material that is flexible and/or stretchable. For example, in at least one such embodiment, the material is comprised of at least one of a silicon-based material, rubber, thermoplastic elastomers, polymeric materials, foils (such as those mixed with epoxy), and various fabrics. Ideally, substrates in direct contact with organic layers will have exceptional barrier capabilities that withstand heat, offer flexibility, have sustained reliability and can be mass produced.

The apparatus **20** further provides an electrophysiological ("EP") module **26** configured for detecting bioelectric signals of the target site **24.** In at least one such embodiment, the at least one EP module **26** is configured as a surface electromyography ("sEMG") sensor in order to detect the electric potential generated by muscles fibers (myocyte). The frequency range of the EMG amplitude is 20µV-5µV; however, other amplitudes may be substituted in further embodiments. When more muscles fibers are recruited to sustain constant loads or to support an increase in load, the amplitude of the sEMG signal increases. In at least one embodiment, the sEMG signal reflects muscular activation driven by the motoneuron, and can be non-invasively collected from skin surface. As an effective tool, sEMG sensor can be used in the diagnosis of neuromuscular diseases, assessment of muscle fatigue and human-machine interface for prosthetic manipulation. In at least one such embodiment, when combining the various modalities of the sEMG sensor with the functionality of the ultrasound module **22,** the apparatus **20** is capable of being used in a wide variety of contexts, including but not limited to: exercise and training; identifying muscles, tendons and other soft tissues injuries; identifying myoelectric manifestations of fatigue; assessing EMG signal modifications in pathologies; evaluating motor coordination and treatment efficacy; identifying neurological diseases; identifying disuse, immobility, and physical inactivity; and measuring neuromuscular alteration due to age. In at least one embodiment, the apparatus **20** is configured for being tightly/closely engaged with the user's skin (so as to eliminate or at least minimize movement artifacts or the displacement of the apparatus **20),** substantially over top of the muscle of interest.

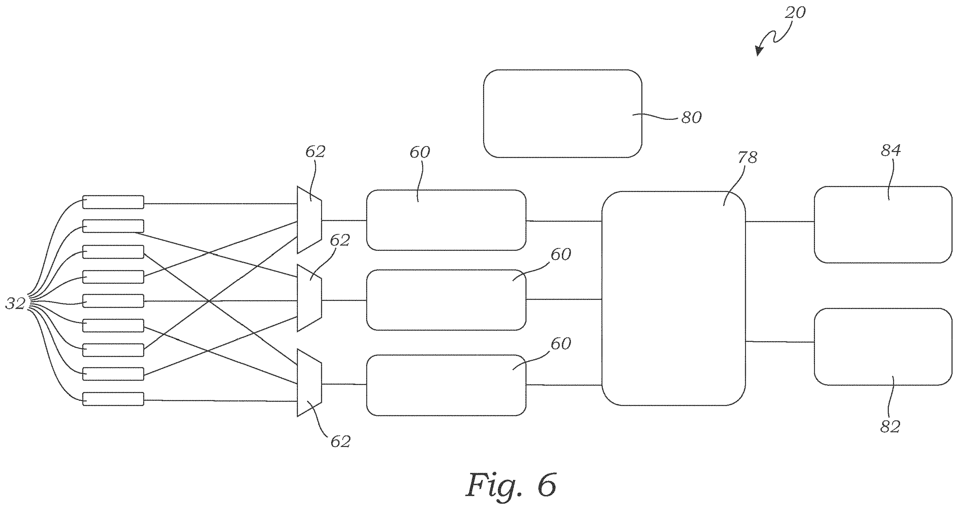

As illustrated in Fig. 6, the apparatus **20** further provides at least one ultrasound transceiver **60** in electrical communication with the at least one piezoelectric sensor **32** of the ultrasound module **22.** In at least one such embodiment, the apparatus **20** provides a relatively greater quantity of piezoelectric sensors **32** than ultrasound transceivers **60,** such that the at least one ultrasound transceiver **60** is in electrical communication with a plurality of piezoelectric sensors **32.** In at least one such embodiment, the apparatus **20** provides at least one analog bidirectional multiplexer **62** in electrical communication with the at least one ultrasound transceiver **60** and the corresponding plurality of piezoelectric sensors **32.** An exemplary configuration of multiplexers **62** is illustrated in the schematic view of Fig. 6. However, it should be noted that the configurations and quantities of the at least one multiplexer **62** as depicted in the drawings (and as described herein) are merely exemplary. In further embodiments, the at least one multiplexer **62** may take on any other configurations (relative to the at least one ultrasound transceiver **60** and the corresponding plurality of piezoelectric sensors **32)** and/or quantities, now known or later developed, so long as the apparatus **20** is capable of substantially carrying out the functionality described herein. In at least one embodiment, where the apparatus **20** provides a plurality of multiplexers **62,** the multiplexers **62** are arranged in a multilayer configuration, with the signal traces between in each layer varying depending on the quantities of multiplexers **62** and piezoelectric sensors **32.** For example, in at least one such embodiment, the quantity of layers is equal to the quantity of multiplexers **62;** while in at least one further such embodiment, the quantity of layers is equal to the quotient of the quantity of piezoelectric sensors **32** divided by the quantity of multiplexers **62.** In still further embodiments, any other quantity of layers, and any other arrangement of signal traces between said layers, may be substituted, so long as the apparatus **20** is capable of substantially carrying out the functionality described herein.

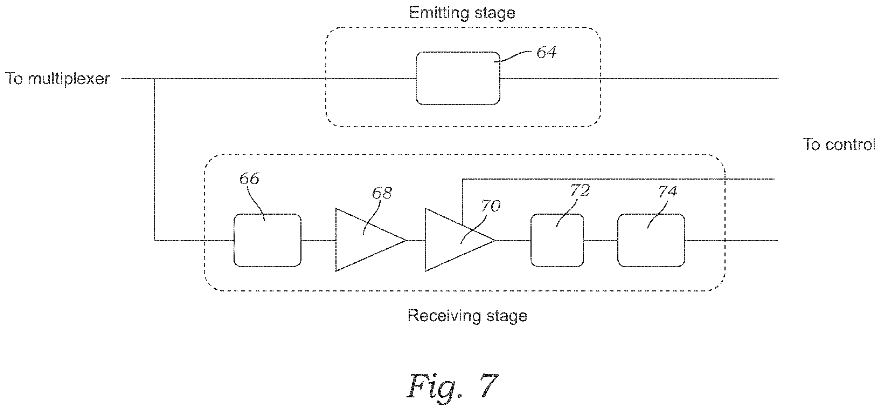

In at least one embodiment, as illustrated in the simplified schematic of Fig. 7, the at least one ultrasound transceiver **60** is itself comprised of a pulser **64,** a transmission/reception switch ("T/R switch") **66,** a low noise amplifier ("LNA") **68,** a variable gain amplifier ("VGA") **70,** a low-pass filter ("LPF") **72,** and an analog-to-digital converter ("ADC") **74.** In a bit more detail, in at least one such embodiment, the at least one ultrasound transceiver **60** is capable of emitting ultrasound pulses with several discrete levels to provide amplitude apodization. In still further embodiments, the at least one ultrasound transceiver **60** may emit pulses with any waveform, and subsequently perform an accurate amplitude apodization which would even include the emission of limited diffraction beams such as the zeroth-order Bessel beam.

Referring again to Fig. 6, the apparatus **20** further provides at least one controller **78** in electrical communication with each of the ultrasound module **22,** EP module **26** and/or NIRS module **28.** Thus, in such embodiments, the controller **78** is configured for interfacing with and managing each of the ultrasound module **22,** EP module **26** and/or NIRS module **28,** and processing the at least one ultrasound image of the target site **24.** In at least one such embodiment, processing may include image reconstruction and/or data compression. In at least one alternate embodiment, one or more of the ultrasound module **22,** EP module **26** and/or NIRS module **28** provide their own dedicated power supply **80.** In at least one embodiment, the controller **78** is also in electrical communication with at least one transceiver **82** configured for transmitting the at least one ultrasound image, and any data associated therewith, to select extemal devices such as computing and electrical devices in communication with the apparatus **20.** In at least one further embodiment, the at least one transceiver **82** is further configured for receiving select information from such external devices as well. The at least one transceiver **82** may utilize any wired- or wireless-based communication protocol (or combination of protocols) now known or later developed, including but not limited to Wi-Fi and Bluetooth-LE.

Additionally, in at least one embodiment, the controller **78** is in selective communication with at least one data storage device **84** (either locally or remotely) configured for storing the at least one ultrasound image and any data associated therewith. It should be noted that the term "data storage device" is intended to include any type of electronic storage medium (or combination of storage mediums) now known or later developed, such as local hard drives, RAM, flash memory, secure digital ("SD") cards, external storage devices, network or cloud storage devices, integrated circuits, etc.

The controller **78** is configured for managing any such further modules (such as the EP module **26** and/or NIRS module **28,** for example). Additionally, the controller is capable of selectively triggering the ultrasound image acquisition upon detection of bioelectric signal and/or specific value of the electrophysiological parameter in the target site **24,** which prevents measurements during irrelevant periods and optimizes the usage of energy intended for ultrasound imaging. Such functionality also allows acquisition at specific instances or when specific events are detected. In at least one such embodiment, the controller **78** is able to optimize the acquisition of ultrasound images of the target site **24** during very fast repetitive movements, even with low acquisition rates - given that the ultrasound image capture process can be synchronized with the periodic movement, the ultrasound images or their lines can be acquired along several periods. In still further embodiments, the controller **78** may be configured to selectively control other aspects and/or functions of the apparatus 20 - such as running the various components in a "low power mode" for example. In at least one embodiment, the controller **78** is at least one of a field-programmable gate array ("FPGA"), a digital signal processor ("DSP"), a microcontroller, and a microprocessor.

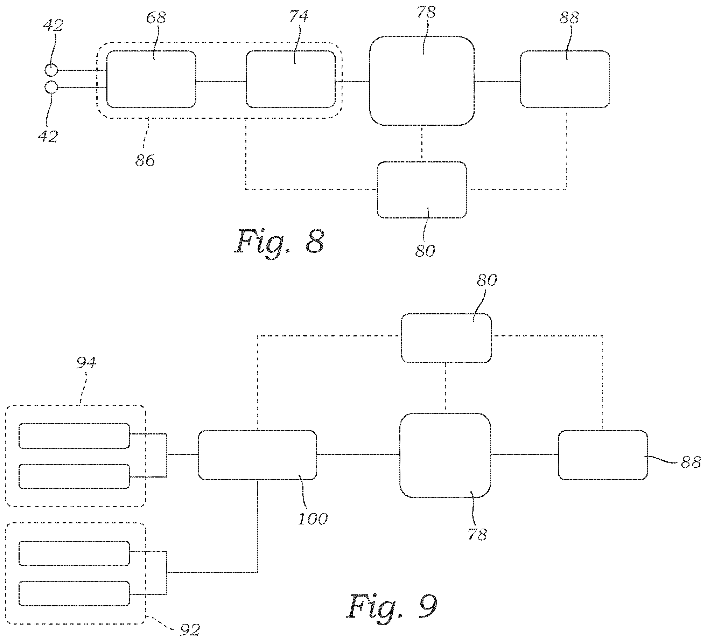

With continued reference to Fig. 6, in at least one embodiment, the apparatus **20** further provides a power supply **80.** The power supply **80** may be any source of power - now known or later developed - capable of providing the necessary power to each of the ultrasound module **22,** EP module **26** and/or NIRS module **28,** including but not limited to one or more batteries (rechargeable or otherwise), an AC adapter, a DC adapter, etc. In at least one alternate embodiment, one or more of the ultrasound module **22,** EP module **26** and/or NIRS module **28** provide their own dedicated power supply **80.**
In at least one embodiment, as illustrated in Fig. 8, the EP module **26** is configured as a multichannel, compact wireless acquisition system, providing at least one electrode **42,** a front-end signal conditioning circuit **86,** a power supply **80,** a controller **78,** and a wireless communication module **88,** such as a Bluetooth-LE module. In at least one embodiment, the EP module **26** provides a biocompatible printed-electrode array to capture bioelectric signals. Additionally, in at least one embodiment, as illustrated in Fig. 1, the EP module provides at least one electrode array comprising 32 electrodes **42** or less (e.g., about 32, 30, 28, 26, 24, 22, 20, 18, 16, 14, 12, 10, 8, 6, 4, 2), along with a reference electrode **90.** However, in further embodiments, any other quantities of electrodes **42** may be utilized. In at least one embodiment, the reference electrode 90 is positioned between two differential electrodes **42** to avoid asymmetry in bioelectric signals recording, and the inter-electrode spacing is increased with this electrode **42** configuration. However, a small inter-electrode spacing is preferable as it will reduce the amount of crosstalk signal detected from adjacent active muscles. Therefore, the inter-electrode spacing are set to be about 32 to 8 millimeters as a preferred compromise (e.g., about 32, 31, 30, 29, 28, 27, 26, 25, 24, 23, 22, 21, 20, 19, 18, 17, 16, 15, 14, 13, 12, 11, 10, 9, 8 mm, or some range therebetween). However, in further embodiments, any other spacing may be utilized, so long as the apparatus **20** is capable of substantially carrying out the functionality described herein. Additionally, in at least one embodiment, the electrodes **42** have a thickness of about 100 micrometers or less (e.g., about 100, 90, 80, 70, 60, 50, 40, 30, 20, 10, 5 µm or less). However, in further embodiments, the electrodes **42** may have any other thickness, so long as the apparatus **20** is capable of substantially carrying out the functionality described herein. The EP module **26** may optionally include electrodes **42** with different sizes and shapes, for example rectangular, circle, oval, ring or disk-shaped electrodes. In a non-limiting example, the electrode **42** array is characterized in that a disk-shaped conductor and at least one ring conductor concentric with the disk-shaped conductor which are disposed on the substrate in order to capture bioelectric signals and configured to provide different weights to the voltages of the conductors generating multiple outputs, corresponding to different sensitivity-based spatial distributions, configured according to requirements for the capture of the bioelectric potentials to be measured. In at least one embodiment, the EP module **26** further provides a coupling layer **48** positioned in contact with a bottom surface of the at least one electrode **42** (or the bottom most one of the at least one electrode **42** array) and configured for selectively adhering the EP module **26** (and, in turn, the apparatus **20)** to the target site **24** - either directly (i.e., adhering to the user's skin) or indirectly (i.e., adhering to a garment or other material that, in turn, is in contact with the user's skin). In at least one embodiment, the coupling layer **48** comprises a gel, (e.g., a hydrogel with adhesive properties). The hydrogel may be electrically conductive and capable of transmitting bioelectric signals between the target site **24** and the EP module **26.**

In at least one embodiment, the electrodes **42** and conductive tracks of the EP module **26** are comprised of conductive metallic inks/pastes produced by using metallic nanoparticles, metal-organic complexes, or metallic salts as precursors (mostly silver-based), conductive polymers, as their conductivity is typically lower than their metallic counterparts but their adhesion and mechanical stability are better, and they do not usually require post-treatment steps. Altematively, dispersions of graphene or CNT's can also be used in printing to create conductive electrodes **42** and/or tracks (conductor patterns). In at least one such embodiment, for example, the electrodes **42** and conductive tracks of the EP module **26** comprise silver polymer paste, stretchable silver conductor paste, medical grade electrically conductive Ag/AgCl ink, or poly(3,4-ethylenedioxythiophene)-poly(styrenesulfonate) due to their flexible processing and durable electrical conductivity.

As mentioned above, the apparatus **20** further provides a near-infrared spectroscopy ("NIRS") module **28** configured for monitoring oxygenation status and/or biochemical measurements of the target site **24.** In at least one such embodiment, as illustrated in Figs. 1 and 9, the at least one NIRS module **28** comprises at least one photodetector **92** and at least one near-infrared light emitting diode ("LED") **94** for muscle oxygenation measurement (muscle oximetry) supported by a substrate.

Human tissues are relatively transparent to light in the near-infrared range between 650-1000 nm. The near-infrared radiation ("NIR") window, also known as the "optical window", is the range of wavelengths that has the maximum depth of penetration in tissue. Indeed, because NIR is minimally absorbed by water and hemoglobin, spectra readings can be easily collected from the body surface, and the main absorbers are blood chromophores of oxygenated hemoglobin (HbO2) and deoxygenated hemoglobin (HHb). When the near-infrared light emitted by the LED **94** passes though the tissue, a portion of light is reflected and absorbed, the remaining light is scattered and can be measured by the at least one photodetector **92.** The depth of the detected NIRS signal can be controlled by the distance between the LED **94** and photodetector **92.** It is generally accepted that, for a source-detector distance of 3 cm, about 1.5 cm (half of the source-detector distance) below the skin surface can be detected through a banana-shaped region. Thus, considering specified anatomies of different muscles, the LED-detector distance can be selected in the range of 2-7 cm for muscle activities detection. However, in further embodiments, any other spacing may be utilized, so long as the apparatus **20** is capable of substantially carrying out the functionality described herein. Moreover, HbO2 and HHb have differential optical absorption properties to the near-infrared light, contracting a muscle changes the amount of near-infrared light that is scattered back to the skin surface, and this change can be detected by the photodetector **92.** By using a modified Beer-Lambert law, relative concentration changes of HbO2 and HHb can be calculated and quantified.

Therefore, combining the advantage of the EP module **26** and NIRS module **28,** in at least one embodiment, would facilitate the understanding of muscle activities from the view of electrophysiology and metabolism, providing more valuable information for human health and physiology performance. For example, NIRS combined with sEMG sensor has been used to obtain more reliable information for assessing metabolic and neuromuscular activity, suggesting the mechanisms of muscle fatigue or injury. However, individual sEMG and NIRS sensor systems were adopted, which resulted in large size, troublesome data synchronism, cumbersome signal wires and limited channels. Thus, the apparatus **20** having integrated EP and NIRS modules **26** and **28** along the ultrasound module **22,** in at least one embodiment, is of great importance to fulfill clinical practical requirements.

In at least one embodiment, one or more of the at least one photodetector **92** may serve to provide a reference signal. For example, the photodetector **92** closest to the near-infrared LED **94** may provide a reference intensity against which the intensities measured by the other photodetectors **92.** In this manner, control over and knowledge of the variations in intensity of the signals emitted by the near-infrared LED **94** may be provided, simplifying the NIRS module **28** design and operation. In at least one embodiment, the photodetectors **92** may be spaced 10 millimeters apart between centers of each adjacent photodetector **92** and between the center of the first photodetector **92** of the plurality of further photodetectors **92** and the near-infrared LED **94.** In other configurations, smaller spacing may be used to allow for inclusion of a greater number of photodetectors **92,** such as a spacing of 8 mm. For example, the photodetectors **92** may be spaced from the near-infrared LED **94** by 8 mm, 16 mm, 24 mm, and 32 mm, respectively. In some configurations, the spacing between neighboring photodetectors **92** may be between 5 mm and 20 mm, less than 5 mm, less than 1 mm or any distance or range of distances within such ranges. In further embodiments, any other spacing may be utilized, so long as the apparatus **20** is capable of substantially carrying out the functionality described herein. In at least one embodiment, the photodetectors **92** are arranged and configured electronically to be operated synchronously with the near-infrared LED **94.** In at least one such embodiment, a photometric front-end **100** is utilized to operating the photodetectors **92** and LED **94.** In at least one embodiment, the near-infrared LED **94** may include a thin light source that may comprise, for example, OLEDs or printable LEDs (organic or inorganic). In at least one such embodiment, the light source comprises a flexible light emitter located between two conductive layers **54** (i.e., electrodes) comprising an anode and a cathode, wherein the flexible light emitter emits light in response to an electric current applied to the anode and cathode. One typical light source uses a transparent substrate, a transparent anode, a flexible light emitter, and a reflective cathode. Light generated from the flexible light emitter is emitted through the transparent anode and transparent substrate. This is commonly referred to as a bottom-emitting light source. By way of example, in at least one such embodiment, a plurality of photodetectors **92** are arranged substantially linearly on a path that originates from the location of the near-infrared LED **94** for measurement of light signals at varying locations from the near-infrared LED **94.** In a preferred configuration, at least two photodetectors **92** are used to measure the intensity of light signals for at least two different distances from the LED **94,** to provide an improved fitting of measured signals as a function of distance with a model used to provide any one or more of oxy(+myo) hemoglobin, (O₂Hb), deoxyhemo(+myo)globin (HHb), total hemo(+myo)globin (tHb) or muscle oxygen saturation (SmO₂) based on the measured intensities.

In at least one embodiment, the conductive layers **54** may comprise a shared electrode such that the same conductive layer **54** serves as a common cathode or as a common anode for the ultrasound module **22,** the EP module **26** and/or the NIRS module **28.** The anode for the EP module **26** and/or the NIRS module **28** comprises, for example, a transparent conductive oxide (TCO), such as, but not limited to, indium tin oxide (ITO), zinc oxide (ZnO), and the like. In practice conductive layers **54** will include a network of tracks connecting these components to their associated electronic systems. Conductive layers **54** additional to those containing the anode or cathode of the ultrasound module **22,** EP module **26** or NIRS module **28** may be included to allow proper routing of all tracks. Moreover conductive layers **54** having a continuous conductive plane may be considered to allow the implementation of impedance-controlled traces (such as microstrip or strip line) and/or provide electromagnetic shielding. Conductive planes or traces may be considered to collect heat generated by any element of the apparatus **20** and conduct it to at least one heat sink where such a heat may be safely transferred to the ambient environment.

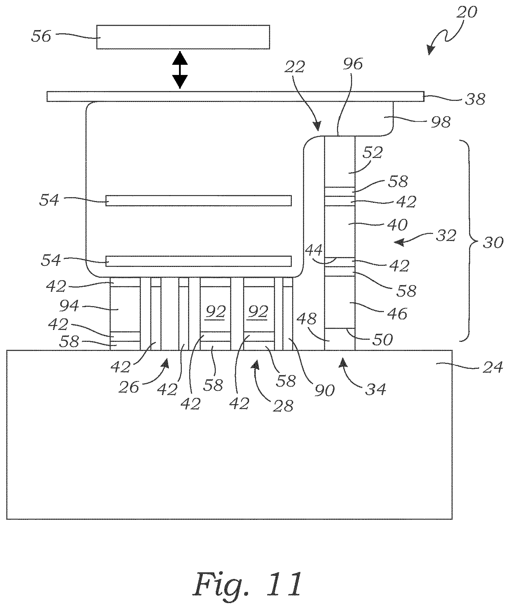

In at least one embodiment, one or more of the electronics system, such as, but not limited to, ultrasound transceiver **60,** analog bidirectional multiplexer **62,** controller **78,** transceiver **82,** power supply **80,** front-end signal conditioning circuit **86,** photometric front-end **100,** wireless communication module **88,** data storage device **84,** and the like, may be contained within at least one microelectronics module **56** (Fig. 1) positioned in contact with a top surface **96** of the at least one backing layer **52.** In at least one further embodiment, the microelectronics module **56** is positioned within a covering **98** positioned on top of and in electrical contact with the various components of the apparatus **20.** In still further embodiments, the microelectronics module **56** may be positioned elsewhere on or relative to the apparatus **20.** For example, in at least one such further embodiment, as illustrated in Fig. 11, the microelectronics module **56** may be positioned external to or separate from the other components of the apparatus **20,** with each of the at least one ultrasound module **22,** EP module **26** and/or NIRS module **28** being in electrical communication with the microelectronics module **56.** Such embodiments allow for fabricating the microelectronics module **56** through traditional and relatively reliable processes while also increasing the modularity of the apparatus **20.** In still further such embodiments, each of the at least one ultrasound module **22,** EP module **26** and/or NIRS module **28** may be implemented together in a single flexible patch (as discussed further below) or separately - dependent at least in part on the technology required in a given use case and the location of the target site **24.**

In at least one embodiment, the covering **98** is constructed out of a transparent or semitransparent material. However, in further embodiments, the covering **98** may be constructed out of an opaque material. The covering **98** may provide comfort for a user, particularly if the user is engaged in physical activity. The covering **98** may provide protection to the various components of the apparatus **20,** keeping dirt and fluid off of the components and providing a cushion to protect the apparatus **20** from impact. The covering **98** may additionally improve the heat transfer between any component of the apparatus **20** and the surrounding environment, provided a reasonably low heat resistivity of the material.

It should be noted that the configuration and arrangement of the various components of the apparatus **20** (including the relative positioning of the components of each of the ultrasound module **22,** EP module **26** and/or NIRS module **28)** as depicted in the drawings is merely exemplary. Accordingly, in further embodiments, the various components may take on any other configurations and arrangements, now known or later developed, so long as the apparatus **20** is capable of substantially carrying out the functionality described herein.

As also illustrated in Fig. 1, in at least one embodiment, the various components of the apparatus **20** described above are configured as a self-contained wearable patch - which can either be adhesively secured to the user's skin (or to a garment in direct contact with the user's skin), or alternatively secured or otherwise integrated into the fabric of a garment that is in direct contact with the user's skin. In each such instance, the apparatus **20** is configured as a wearable, flexible solution for providing remote ambulatory monitoring of the target site. Thus, in embodiments where the apparatus **20** is utilized in the context of MSK ultrasounds (which are normally used to generate ultrasound images of muscles, tendons, ligaments and joints throughout the body, helping to diagnose sprains, strains, tears, and other soft tissue conditions), the apparatus **20** will allow real-time data collection in the sports medicine and real-time healthcare monitoring domains, while also being useful in a multitude of advanced applications, including human-machine interfaces, advanced prosthetic technologies (bionics), electronic skins, wearable consumer electronics and soft robotics, to name a few. Furthermore, by incorporating each of the ultrasound module **22,** EP module **26** and NIRS module **28** in at least one embodiment, the apparatus **20** is capable of functioning as a novel multi-modal "3-in-1" system (or at least a "2-in-1" system, where only one of the EP module **26** or NIRS module **28** are incorporated with the ultrasound module **22)** that simultaneously acquires ultrasonic imaging, bioelectric signals and oxygenation status and/or biochemical measurements - including but not limited to sonomyography ("SMG"), electromyography ("EMG"), electrocardiography ("ECG"), electroencephalography ("EEG"), galvanic skin response ("GSR"), photoplethysmography ("PPG"), arterial oxygen saturation ("Sp02"), oxy(+myo) hemoglobin, (O₂Hb), deoxyhemo(+myo)globin (HHb), total hemo(+myo)globin (tHb), muscle oxygen saturation (SmO₂), muscle activity, emotions, arterial saturation of carbon monoxide ("SpCO") and blood carbon dioxide ("CO2"), blood pressure ("BP"), respiration, such as respiration frequency ("RF") and/or respiration volume ("RV"), heart rate ("HR") and/or heart rate variability ("HRV"), pulse, bioimpedance, and temperature, such as skin temperature ("ST"), and/or core body temperature - in various biomedical and clinical applications.

Aspects of the present invention are defined in the appended claims.

In closing, regarding the exemplary embodiments of the present invention as shown and described herein, it will be appreciated that a wearable ultrasound apparatus is disclosed and configured for use in connection with various biomedical applications, including musculoskeletal ("MSK") imaging and analysis. Because the principles of the invention may be practiced in a number of configurations beyond those shown and described, it is to be understood that the invention is not in any way limited by the exemplary embodiments, but is generally directed to a wearable ultrasound apparatus and is able to take numerous forms in accordance with the appended claims without departing from the scope of the invention. It will also be appreciated by those skilled in the art that the present invention is not limited to the particular geometries and materials of construction disclosed, but may instead entail other functionally comparable structures or materials, now known or later developed, without departing from the scope of the invention.

Certain embodiments of the present invention are described herein, including the best mode known to the inventor(s) for carrying out the invention. Of course, variations on these described embodiments will become apparent to those of ordinary skill in the art upon reading the foregoing description. The inventor(s) expect skilled artisans to employ such variations as appropriate, and the inventor(s) intend for the present invention to be practiced otherwise than specifically described herein. Moreover, any combination of the above-described embodiments in all possible variations thereof is encompassed by the invention unless otherwise indicated herein or otherwise clearly contradicted by context.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein. Similarly, as used herein, unless indicated to the contrary, the term "substantially" is a term of degree intended to indicate an approximation of the characteristic, item, quantity, parameter, property, or term so qualified, encompassing a range that can be understood and construed by those of ordinary skill in the art.

Use of the terms "may" or "can" in reference to an embodiment or aspect of an embodiment also carries with it the alternative meaning of "may not" or "cannot." As such, if the present specification discloses that an embodiment or an aspect of an embodiment may be or can be included as part of the inventive subject matter, then the negative limitation or exclusionary proviso is also explicitly meant, meaning that an embodiment or an aspect of an embodiment may not be or cannot be included as part of the inventive subject matter. In a similar manner, use of the term "optionally" in reference to an embodiment or aspect of an embodiment means that such embodiment or aspect of the embodiment may be included as part of the inventive subject matter or may not be included as part of the inventive subject matter. Whether such a negative limitation or exclusionary proviso applies will be based on whether the negative limitation or exclusionary proviso is recited in the claimed subject matter.

The terms "a," "an," "the" and similar references used in the context of describing the present invention (especially in the context of the following claims) are to be construed to cover both the singular and the plural, unless otherwise indicated herein or clearly contradicted by context. Further, ordinal indicators - such as "first," "second," "third," etc. - for identified elements are used to distinguish between the elements, and do not indicate or imply a required or limited number of such elements, and do not indicate a particular position or order of such elements unless otherwise specifically stated. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

When used in the claims, whether as filed or added per amendment, the open-ended transitional term "comprising" (along with equivalent open-ended transitional phrases thereof such as "including," "containing" and "having") encompasses all the expressly recited elements, limitations, steps and/or features alone or in combination with un-recited subject matter; the named elements, limitations and/or features are essential, but other unnamed elements, limitations and/or features may be added and still form a construct within the scope of the claim. Specific embodiments disclosed herein may be further limited in the claims using the closed-ended transitional phrases "consisting of' or "consisting essentially of" in lieu of or as an amendment for "comprising." When used in the claims, whether as filed or added per amendment, the closed-ended transitional phrase "consisting of' excludes any element, limitation, step, or feature not expressly recited in the claims. The closed-ended transitional phrase "consisting essentially of' limits the scope of a claim to the expressly recited elements, limitations, steps and/or features and any other elements, limitations, steps and/or features that do not materially affect the basic and novel characteristic(s) of the claimed subject matter. Thus, the meaning of the open-ended transitional phrase "comprising" is being defined as encompassing all the specifically recited elements, limitations, steps and/or features as well as any optional, additional unspecified ones. The meaning of the closed-ended transitional phrase "consisting of' is being defined as only including those elements, limitations, steps and/or features specifically recited in the claim, whereas the meaning of the closed-ended transitional phrase "consisting essentially of' is being defined as only including those elements, limitations, steps and/or features specifically recited in the claim and those elements, limitations, steps and/or features that do not materially affect the basic and novel characteristic(s) of the claimed subject matter. Therefore, the open-ended transitional phrase "comprising" (along with equivalent open-ended transitional phrases thereof) includes within its meaning, as a limiting case, claimed subject matter specified by the closed-ended transitional phrases "consisting of' or "consisting essentially of." As such, embodiments described herein or so claimed with the phrase "comprising" are expressly or inherently unambiguously described, enabled and supported herein for the phrases "consisting essentially of" and "consisting of."

It should be understood that the logic code, programs, modules, processes, methods, and the order in which the respective elements of each method are performed are purely exemplary. Depending on the implementation, they may be performed in any order or in parallel, unless indicated otherwise in the present disclosure. Further, the logic code is not related, or limited to any particular programming language, and may comprise one or more modules that execute on one or more processors in a distributed, non-distributed, or multiprocessing environment. Additionally, the various illustrative logical blocks, modules, methods, and algorithm processes and sequences described in connection with the embodiments disclosed herein can be implemented as electronic hardware, computer software, or combinations of both. To clearly illustrate this interchangeability of hardware and software, various illustrative components, blocks, modules, and process actions have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of this document.

The phrase "non-transitory," in addition to having its ordinary meaning, as used in this document means "enduring or long-lived." The phrase "non-transitory computer readable medium," in addition to having its ordinary meaning, includes any and all computer readable mediums, with the sole exception of a transitory, propagating signal. This includes, by way of example and not limitation, non-transitory computer-readable mediums such as register memory, processor cache and random-access memory ("RAM").

The methods as described above may be used in the fabrication of integrated circuit chips. The resulting integrated circuit chips can be distributed by the fabricator in raw wafer form (that is, as a single wafer that has multiple unpackaged chips), as a bare die, or in a packaged form. In the latter case, the chip is mounted in a single chip package (such as a plastic carrier, with leads that are affixed to a motherboard or other higher level carrier) or in a multi-chip package (such as a ceramic carrier that has either or both surface interconnections or buried interconnections). In any case, the chip is then integrated with other chips, discrete circuit elements, and/or other signal processing devices as part of either (a) an intermediate product, such as a motherboard, or (b) an end product. The end product can be any product that includes integrated circuit chips, ranging from toys and other low-end applications to advanced computer products having a display, a keyboard or other input device, and a central processor.

## Claims

1. A wearable ultrasound apparatus (20)
positionable on a target site of a user's body and
comprising:
at least one ultrasound module (22) configured for obtaining at least one ultrasound image of the target site, the ultrasound module comprising:
at least one ultrasound transducer (30) positioned on at least one resilient substrate (38), the at least one ultrasound transducer comprising at least one sensor;
at least one conductive layer (54) positioned in electrical communication with the at least one sensor; and at least one ultrasound transceiver (60) in electrical communication with the at least one sensor;
at least one electrophysiological, EP, module (26) positioned on the at least one resilient substrate and configured for detecting bioelectric signals of the target site; and at least one controller (78)
in electrical communication with each of the ultrasound module and EP module via the conductive layers, the at least one controller configured for selectively causing the ultrasound module to obtain at least one ultrasound image of the target site upon detection of bioelectric signal in the target site via the EP module; and
a near-infrared spectroscopy, NIRS, module (28) positioned on the at least one resilient substrate, in electrical communication with the at least one controller, and configured for monitoring oxygenation status and/or biochemical measurements of the target site.

2. The wearable ultrasound apparatus of claim 1, wherein the at least one sensor is at least one of a piezoelectric or a microelectromechanical, MEM, sensor.

3. The wearable ultrasound apparatus of claim 2, wherein the at least one sensor is a piezoelectric sensor comprising a piezoelectric material sandwiched between two or more electrodes.

4. The wearable ultrasound apparatus of claim 1, wherein the at least one ultrasound module further comprises at least one matching layer positioned on a bottom surface of the at least one sensor and configured for providing acoustic impedance adaption.

5. The wearable ultrasound apparatus of claim 1, wherein the ultrasound module further comprises at least one backing layer positioned in contact with a top surface of the at least one sensor furthest from the target site, the at least one backing layer configured for absorbing any ultrasound waves radiated by the at least one sensor that are not directed toward the target site.

6. The wearable ultrasound apparatus of claim 1, wherein the at least one ultrasound module further comprises at least one encapsulation layer positioned to isolate the at least one conductive layer from the surrounding environment.

7. The wearable ultrasound apparatus of claim 1, further comprising a coupling layer positioned in contact with a bottom surface of a bottom most one of the at least one ultrasound transducer and configured for selectively adhering the apparatus to the target site.

8. The wearable ultrasound apparatus of claim 7, wherein the coupling layer has an acoustic impedance that approximates an acoustic impedance of the target site so as to provide impedance matching.

9. The wearable ultrasound apparatus of claim 1, wherein the at least one ultrasound transducer comprises
a plurality of adjacently arranged sensors configured as at least one array.

10. The wearable ultrasound apparatus of claim 9, wherein the at least one ultrasound transducer comprises a plurality of arrays positioned in a side-by-side arrangement.

11. The wearable ultrasound apparatus of claim 9, wherein the at least one ultrasound transducer comprises a plurality of arrays arranged so as to acquire orthogonal cross-sections of the target site.

12. The wearable ultrasound apparatus of claim 9, wherein the at least one array is configured as a curve.

13. The wearable ultrasound apparatus of claim 9, wherein a subset of contiguous sensors are configured for being simultaneously activated on demand.

14. The wearable ultrasound apparatus of claim 9, wherein a plurality of sensors are sandwiched between a plurality of electrodes arranged in a row-column configuration so as to form at least one quasi-two-dimensional array.

15. The wearable ultrasound apparatus of claim 1, wherein the apparatus comprises a relatively greater quantity of sensors than ultrasound transceivers, such that the at least one ultrasound transceiver is in electrical communication with a plurality of sensors.

16. The wearable ultrasound apparatus of claim 15, further comprising at least one bidirectional multiplexer in electrical communication with the at least one ultrasound transceiver and the corresponding plurality of sensors.

17. The wearable ultrasound apparatus of claim 1, wherein the EP module comprises at least one electrode array, in electrical communication with the at least one signal conditioning circuit, and configured for detecting bioelectric signals of the target site.

## Patentansprüche

1. Ein tragbarer Ultraschallapparat (20), der an einer Zielstelle des Körpers eines Nutzers angebracht werden kann und Folgendes umfasst:
mindestens ein Ultraschallmodul (22), konfiguriert zum Aufnehmen mindestens eines Ultraschallbildes der Zielstelle, das Ultraschallmodul umfassend:
mindestens einen Ultraschallwandler (30), der auf mindestens einem belastbaren Substrat (38) positioniert ist, der mindestens eine Ultraschallwandler umfassend mindestens einen Sensor;
mindestens eine leitfähige Schicht (54), die in elektrischer Verbindung mit dem mindestens einen Sensor positioniert ist; und
mindestens einen Ultraschallempfänger (60) in elektrischer Verbindung mit dem mindestens einen Sensor;
mindestens ein elektrophysiologisches, EP-, Modul (26), das auf dem mindestens einen belastbaren Substrat positioniert und zur Detektion bioelektrischer Signale der Zielstelle konfiguriert ist; und
mindestens einen Kontroller (78) in elektrischer Verbindung mit sowohl dem Ultraschallmodul als auch dem EP-Modul über die leitfähigen Schichten, wobei der mindestens eine Kontroller konfiguriert ist, selektiv zu verursachen, dass das Ultraschallmodul mindestens ein Ultraschallbild der Zielstelle nach Detektion von bioelektrischem Signal in der Zielstelle über das EP-Modul aufnimmt; und
mindestens ein Nahinfrarotsprektroskopie-, NIRS-, Modul (29), das auf dem mindestens einen belastbaren Substrat positioniert ist, in elektrischer Verbindung mit dem mindestens einen Kontroller, und konfiguriert zur Überwachung des Sauerstoffsättigungsstatus und/oder biochemischer Messungen der Zielstelle.

2. Der tragbare Ultraschallapparat aus Anspruch 1, wobei der mindestens eine Sensor mindestens eines von einem piezoelektrischen oder einem mikroelektromeschanischen, MEM-, Sensor ist.

3. Der tragbare Ultraschallapparat aus Anspruch 2, wobei der mindestens eine Sensor ein piezoelektrischer Sensor umfassend ein zwischen zwei oder mehr Elektroden liegendes piezoelektrisches Material ist.

4. Der tragbare Ultraschallapparat aus Anspruch 1, wobei das mindestens eine Ultraschallmodul weiter mindestens eine übereinstimmende Schicht umfasst, die an einer unteren Oberfläche des mindestens einen Sensors positioniert ist und konfiguriert ist, akustische Impendanzanpassung bereitzustellen.

5. Der tragbare Ultraschallapparat aus Anspruch 1, wobei das Ultraschallmodul weiter mindestens eine hintere Schicht umfasst, die in Kontakt mit einer oberen Oberfläche des mindestens einen Sensors am weitesten von der Zielstelle positioniert ist, wobei die mindestens eine hintere Schicht konfiguriert ist zur Absorption jeglicher von dem mindestens einen Sensor abgestrahlter Ultraschallwellen, die nicht zur Zielstelle gerichtet sind.

6. Der tragbare Ultraschallapparat aus Anspruch 1, wobei das mindestens eine Ultraschallmodul weiter mindestens eine Verkapselungsschicht umfasst, die positioniert ist, die mindestens eine leitfähige Schicht von der Umgebung zu isolieren.

7. Der tragbare Ultraschallapparat aus Anspruch 1, weiter umfassend eine Kopplungsschicht, die in Kontakt mit einer unteren Oberfläche eines untersten des mindestens einen Ultraschallwandlers positioniert ist und konfiguriert ist, den Apparat selektiv an der Zielstelle anzuheften.

8. Der tragbare Ultraschallapparat aus Anspruch 7, wobei die Kopplungsschicht eine akustische Impendanz hat, die sich einer akustischen Impendanz der Zielstelle annähert, so dass Impendanzanpassung bereitgestellt wird.

9. Der tragbare Ultraschallapparat aus Anspruch 1, wobei der mindestens eine Ultraschallwandler eine Vielzahl benachbart angeordneter Sensoren umfasst, die als mindestens eine Anordnung konfiguriert sind.

10. Der tragbare Ultraschallapparat aus Anspruch 9, wobei der mindestens eine Ultraschallwandler eine Vielzahl von Seite an Seite angeordneten Anordnungen umfasst.

11. Der tragbare Ultraschallapparat aus Anspruch 9, wobei der mindestens eine Ultraschallwandler eine Vielzahl von so angeordneten Anordnungen umfasst, dass orthogonale Querschnitte der Zielstelle aufgenommen werden.

12. Der tragbare Ultraschallapparat aus Anspruch 9, wobei die mindestens eine Anordnung als eine Kurve konfiguriert ist.

13. Der tragbare Ultraschallapparat aus Anspruch 9, wobei ein Teilsatz von zusammenhängenden Sensoren konfiguriert ist, auf Anfrage gleichzeitig aktiviert zu werden.

14. Der tragbare Ultraschallapparat aus Anspruch 9, wobei eine Vielzahl von Sensoren zwischen einer Vielzahl von Elektroden positioniert sind, die in einer Zeilen-Spalten-Konfiguration angeordnet sind, so dass sie mindestens eine quasi-zweidimensionale Anordnung bilden.

15. Der tragbare Ultraschallapparat aus Anspruch 1, wobei der Apparat eine relativ größere Menge an Sensoren als Ultraschallempfänger umfasst, so dass der mindestens eine Ultraschallempfänger in elektrischer Verbindung mit einer Vielzahl von Sensoren ist.

16. Der tragbare Ultraschallapparat aus Anspruch 15, weiter umfassend mindestens einen bidirektionalen Multiplexer in elektrischer Verbindung mit dem mindestens einen Ultraschallempfänger und der entsprechenden Vielzahl von Sensoren.

17. Der tragbare Ultraschallapparat aus Anspruch 1, wobei das EP-Modul mindestens eine Elektrodenanordnung umfasst, in elektrischer Verbindung mit dem mindestens einen Signalkonditionierungsschaltkreis und konfiguriert zur Detektion bioelektrischer Signale der Zielstelle.

## Revendications

1. Appareil à ultrasons portable (20) positionnable sur un site cible du corps d'un utilisateur et comprenant :
au moins un module à ultrasons (22) configuré pour obtenir au moins une image ultrasonore du site cible, le module à ultrasons comprenant :
au moins un transducteur à ultrasons (30) positionné sur au moins un substrat élastique (38), l'au moins un transducteur à ultrasons comprenant au moins un capteur ;
au moins une couche conductrice (54) positionnée en communication électrique avec l'au moins un capteur ; et
au moins un émetteur-récepteur à ultrasons (60) en communication électrique avec l'au moins un capteur ;
au moins un module électrophysiologique, EP, (26) positionné sur l'au moins un substrat élastique et configuré pour détecter des signaux bioélectriques du site cible ; et
au moins un dispositif de commande (78) en communication électrique avec chacun du module à ultrasons et du module EP par l'intermédiaire des couches conductrices, l'au moins un dispositif de commande étant configuré pour amener sélectivement le module à ultrasons à obtenir au moins une image ultrasonore du site cible lors de la détection d'un signal bioélectrique dans le site cible par l'intermédiaire du module EP ; et
un module de spectroscopie proche infrarouge, NIRS, (28) positionné sur l'au moins un substrat élastique, en communication électrique avec l'au moins un dispositif de commande, et configuré pour surveiller l'état d'oxygénation et/ou des mesures biochimiques du site cible.

2. Appareil à ultrasons portable selon la revendication 1, dans lequel l'au moins un capteur est au moins l'un parmi un capteur piézoélectrique ou un capteur microélectromécanique, MEM.

3. Appareil à ultrasons portable selon la revendication 2, dans lequel l'au moins un capteur est un capteur piézoélectrique comprenant un matériau piézoélectrique pris en sandwich entre deux électrodes ou plus.

4. Appareil à ultrasons portable selon la revendication 1, dans lequel l'au moins un module à ultrasons comprend en outre au moins une couche de correspondance positionnée sur une surface inférieure de l'au moins un capteur et configurée pour assurer une adaptation d'impédance acoustique.

5. Appareil à ultrasons portable selon la revendication 1, dans lequel le module à ultrasons comprend en outre au moins une couche de support positionnée au contact d'une surface supérieure de l'au moins un capteur le plus éloigné du site cible, l'au moins une couche de support étant configurée pour absorber toutes les ondes ultrasonores rayonnées par l'au moins un capteur qui ne sont pas dirigées vers le site cible.

6. Appareil à ultrasons portable selon la revendication 1, dans lequel l'au moins un module à ultrasons comprend en outre au moins une couche d'encapsulation positionnée pour isoler l'au moins une couche conductrice de l'environnement ambiant.

7. Appareil à ultrasons portable selon la revendication 1, comprenant en outre une couche de couplage positionnée au contact d'une surface inférieure d'un transducteur à ultrasons situé le plus bas parmi l'au moins un transducteur à ultrasons et configurée pour faire sélectivement adhérer l'appareil au site cible.

8. Appareil à ultrasons portable selon la revendication 7, dans lequel la couche de couplage présente une impédance acoustique qui s'approche d'une impédance acoustique du site cible de manière à assurer une correspondance d'impédance.

9. Appareil à ultrasons portable selon la revendication 1, dans lequel l'au moins un transducteur à ultrasons comprend
une pluralité de capteurs agencés de manière adjacente configurés sous forme d'au moins un réseau.

10. Appareil à ultrasons portable selon la revendication 9, dans lequel l'au moins un transducteur à ultrasons comprend une pluralité de réseaux positionnés dans un agencement côte à côte.

11. Appareil à ultrasons portable selon la revendication 9, dans lequel l'au moins un transducteur à ultrasons comprend une pluralité de réseaux agencés de manière à acquérir des sections transversales orthogonales du site cible.

12. Appareil à ultrasons portable selon la revendication 9, dans lequel l'au moins un réseau est configuré sous forme de courbe.

13. Appareil à ultrasons portable selon la revendication 9, dans lequel un sous-ensemble de capteurs contigus est configuré pour être activé simultanément à la demande.

14. Appareil à ultrasons portable selon la revendication 9, dans lequel une pluralité de capteurs sont pris en sandwich entre une pluralité d'électrodes agencées dans une configuration rangée-colonne de manière à former au moins un réseau quasi bidimensionnel.

15. Appareil à ultrasons portable selon la revendication 1, dans lequel l'appareil comprend une quantité relativement plus importante de capteurs que d'émetteurs-récepteurs à ultrasons, de sorte que l'au moins un émetteur-récepteur à ultrasons soit en communication électrique avec une pluralité de capteurs.

16. Appareil à ultrasons portable selon la revendication 15, comprenant en outre au moins un multiplexeur bidirectionnel en communication électrique avec l'au moins un émetteur-récepteur à ultrasons et la pluralité correspondante de capteurs.

17. Appareil à ultrasons portable selon la revendication 1, dans lequel le module EP comprend au moins un réseau d'électrodes, en communication électrique avec l'au moins un circuit de conditionnement de signal, et configuré pour détecter des signaux bioélectriques du site cible.
